Europäisches Patentamt

(19) European Patent Office

Office européen des brevets

(11) Publication number: **0 028 251**
**B1**

(12) **EUROPEAN PATENT SPECIFICATION**

(45) Date of publication of patent specification: **16.03.83**

(51) Int. Cl.³: **C 07 B 19/02**

(21) Application number: **80901093.7**

(22) Date of filing: **28.04.80**

(86) International application number:
**PCT/US80/00488**

(87) International publication number:
**WO 80/02421 13.11.80 Gazette 80/26**

(54) Process for producing N-acyl-D-phenylalanine ester.

(30) Priority: **08.05.79 US 37162**

(43) Date of publication of application:
**13.05.81 Bulletin 81/19**

(45) Publication of the grant of the patent:
**16.03.83 Bulletin 83/11**

(84) Designated Contracting States:
**AT CH DE FR GB LI LU NL SE**

(56) References cited:
BE - A - 855 051
FR - A - 2 380 251
US - A - 3 031 380
US - A - 3 347 752
US - A - 3 813 317
US - A - 3 878 043
US - A - 3 907 638
US - A - 3 963 573

(73) Proprietor: **ETHYL CORPORATION**
**330 South Fourth Street P.O. Box 2189**
**Richmond Virginia 23219 (US)**

(72) Inventor: **BAUER, Dennis P.**
**3650 Nicholson Drive, Apartment 2179**
**Baton Rouge, LA 70802 (US)**

(74) Representative: **Baillie, Iain Cameron et al,**
**c/o Ladas & Parry Isartorplatz 5**
**D-8000 München 2 (DE)**

Courier Press, Leamington Spa, England

(56) References cited:

CHEMICAL ABSTRACTS, vol. 76, no. 15, 10th
April 1972, page 170, no. 82748f
Columbus, Ohio, U.S.A.
T.N. PATTABIRAMAN et al.: "Comparative
studies of the specificities of alpha-chromotrypsin
and subtilisin BNP'. Flexible substrates" &
BIOCHEM. J. 1972, 126 (3), 645—657

CHEMICAL ABSTRACTS, vol. 88, no. 15, 10th
April 1978, page 611, no. 105736q
Columbus, Ohio, U.S.A.
CHI-HEUY WONG et al.: "The resolution of
amino acids by enzymes. Part I: Proteolytic
enzyme bromelain for hydrolysis of L-amino acid
methyl esters" & J. CHIN. CHEM. SOC. (TAIPEI)
1977, 24(3), 129—133

C. R. TRAV. ALB. CARLSBERG SER. CHIM. 29
36—48 (1954)

BIOCHEMISTRY, VOL. 3 NO. 12 PP. 1861—74
(1964)

JOURNAL OF BIOLOGICAL CHEMISTRY VOL.
241 NO. 24 PP. 5974—5976 12 DECEMBER
1964

JOURNAL OF BIOCHEMISTRY VOL. 62 NO. 6
PP. 633—41 (1967)

AGR. BIOL. CHEM., VOL. 31 NO. 10 PP.
1151—1158 (1967)

JOURNAL OF BIOLOGICAL CHEMISTRY VOL.
243 NO. 7 PP. 1344—1348, 10 APRIL 1968

AGR. BIOL. CHEM. VOL. 34 NO. 9 PP.
1383—1392 (1970)

SYNTHETIC PRODUCTION AND UTILIZATION
OF AMINO ACIDS, J. WILEY & SONS PP.
171—179 (1974)

Process for producing N-acyl-D-phenylalanine ester

This invention relates in part to a process for producing N-acyl-D-phenylalanine ester. In its most general form, the invention deals with the resolution of certain racemic mixtures of derivatives of phenylalanine with a high degree of effectiveness. Moreover, as will appear herein, the invention in certain applications comprises plural-stage operations whereby racemic D,L-phenylalanine supplies can be converted into either desired optical isomeric form.

The resolution of racemic D,L-phenylalanine mixtures has heretofore been disclosed. Thus a first method involes asymmetric hydrolysis of N-chloroacetyl-D,L-phenylalanine by the carboxypeptidase in pancrease. A second involves asymmetric hydrolysis of N-acetyl-D,L-phenylalanine by mould aminoacylase which process can be operated in a continuous fashion using a packed column. And, finally, a physico-chemical resolution based on preferential crystallization of isomers from supersaturated solution of acetyl-D,L-phenylalanine ammonium salt. In addition, there are several synthetic methods for production of L-phenylalanine. For example, conversion of L-tyrosine, treatment of synthetic phenylpyruvic acid with transaminase and enzymatic isomerization of synthetic D,L-phenylalanine by microorganisms. Kaneko et al, *Synthetic Production and Utilization of Amino Acids,* J. Wiley & Sons, pp. 171—179 (1974).

In Belgian Patent 855,051, there is taught a method and a composition for treatment of D,L-amino acids to resolve the racemic mixture and obtain L-isomer in which a supported aminoacylase is used to treat aqueous solutions of the D,L-amino acid. The support is a porous inorganic substrate of specified grain size, surface area pore diameter and volume and is coated with a network polymer film, or carries a tertiary amine or quaternary ammonium salt group. Typical supports are titania, alumina or silica. The network polymer can be polyamino epoxides, polyamine-formaldehyde, phenolformaldehyde mixtures and polymerized mixtures of vinyl monomers. The amino-acylases are enzymes of animal origin, such as pork kidney extracts or microorganism products, such as from *Aspergillus, Lactobacillus arabinosus, Micrococcus glutamicus,* and *Pseudomonoas cruciviae.* The process includes contacting the amino acids, such as N-acetyl-D,L-amino acid, with the complex of support/tertiary amine/polymer network/enzyme, for example by passing through a packed column.

In U.S. 3,963,573, there is taught a process for producing optically pure N-acyl-L-methionine by subjecting an N-acyl-D,L-methionine ester to the action of a proteolytic enzyme selected from the group consisting of sulfhydryl proteinases and microbially derived serine proteinases and separating the resulting N-acyl-L-methionine. The art has recognized that certain proteolytic enzymes can be produced in a reproducible form, such as from *Bacillus subtilis, Guntelberg Trav. Lab. Carlsberg,* Ser. Chim. Vol. *29,* p. 36 (1954). The proteolytic enzyme prepared from the strain of Bacillus subtilis was purified by crystallization and its physico-chemical properties were determined. The enzymatic properties were investigated insofar as optimum pH for milk coagulation, stability, degradation of casein, hydrolysis of hemoglobin, activators and inhibitors for the enzymes, the effect on ovalbumin and other characteristics. In the *Journal of Biological Chemistry,* Vol. 23, No. 7, pp.1344—1348 (1968), Barel, examined the activity of Carlsberg and Novo subtilisins toward a number of N-acetylamino acid esters and amino acid esters. The enzymes were also compared with respect to their efficiency in catalyzing aminolysis reactions, their rates of inactivation by certain aromatic sulfonyl halides and the rates of deacylation of their N-trans-cinnamoyl derivatives. Although the enzymes were found qualitatively indistinguishable from the standpoint of substrate specificity, significant quantitative differences were observed. Thus, the microbially derived serine proteinases, for example, Novo and Carlsberg subtilisin exhibited varying of degrees of esterage activity on various N-acyl-L-amino acid esters. However, there is not a process for resolution of N-acyl-D,L-phenylalanine esters employing the activity of serine proteinases.

U.S. Patent 3 878 043 discloses a process for separation of the D and L isomers of N-acyl-dihydroxyphenyl-alanine or N-acyl-hydroxyal-koxyphenyl-alanine by selective hydrolysis of ester derivatives thereof with use of a proteolitic enzyme. The preferred enzyme is $\alpha$-chymo-tripsin which is the only enzyme exemplified, and is not a microbially derived serine proteinase.

French patent publication 2 380 251 discloses a process for separation of N-acyl-D,L-threonine by selective hydrolysis of its methyl ester with use of a proteolitic enzyme which is a microbially derived serine proteinase.

Until recent periods there has not been an urgent need for the isolated optically pure isomers of phenylalanine. It is now known, however, that D-phenylalanine is a simple non-addictive analgesic compound that stimulates the body's own pain-fighting system and is capable of giving significant relief and long-term analgesia for chronic pain patients.

With respect to L-phenylalanine, U.S. patent 3,901,871 discloses a valuable utility of the L-form as an intermediate in the manufacture of the sweetening agent methyl-$\alpha$-L-aspartyl-L-phenylalanate. In U.S. patent 4,151,198 is described a process of resolving racemic mixtures of N-acyl-D,L-phenylalanine, said process involving reacting with D(-)-2-(2,5-dimethyl-benzylamino)-1-butanol, and crystallizing from a solution of the reaction product on L-isomer, which is converted to the L-phenylalanine.

The prior techniques which have been disclosed resolving phenylalanine antipodes (or derivative compound mixtures) which of interest, appear to be complex and probably non-viable as practical or commercial possibilities. It is, therefore, quite apparent that a need has existed for achieving a high level of separation of D,L-phenylalanine or derivative compounds into the optical isomers of adequate purity.

As indicated above, it has been recognized that certain enzymes do act with some selectivity on optical isomer mixtures of amino acid sources, specifically some acyl amino acid esters. Such an approach, however, is found to be of non-predictable efficacy with respect to the various amino acid structures and available enzyme materials. As is shown by comparative data herein, non-foreseeable variations exist which particularly bear on the criteria of performance which would be controlling with respect to industrial applicability.

Despite the non-predictability of effective performance, it has been found that selective enzymatic action can be accomplished on a racemic mixture of derivatives of phenylalanine. As is shown herein, it is found that a highly selective reaction can be effected of essentially only the N-acetyl-L-phenylalanine methyl ester in admixture with the corresponding N-acetyl-D-phenylalanine methyl ester.

The present invention meets the needs outlined above. A key step of the invention comprises

a) subjecting an aqueous solution of mixture of N-acetyl-D-phenylalanine methyl ester and N-acetyl-L-phenylalanine methyl ester to the action of a proteolytic enzyme which is a member selected from the group consisting of the microbially derived serine proteinases, at a pH in the range of from 5 to 10;

b) separating the resulting N-acetyl-L-phenylalanine from the unreacted N-acyl-D-phenylalanine ester; and

c) recovering the N-acyl-D-phenylalanine ester.

In certain elaborations of the invention, an overall process is established comprising

a) providing a mixture of N-acetyl-D,L-phenylalanine methyl esters;

b) separating said mixture by the selective treatment as expressed above into N-acetyl-L-phenylalanine and N-acetyl-D-phenylalanine methyl ester and recovering said compounds; and

c) converting said compounds if desired to the D and L phenylalanines, respectively.

To the foregoing sequence can be added, dependent on specific market needs, the conversion of either of the separated optical isomers by known techniques into a racemic mixture, which is readily convertible to the feed stock for step a) above. It is thus apparent that such embodiments provide, in substance, for the transformation of a racemic mixture of D,L-phenylalanine into either compound.

It has been found that certain readily available proteinases, namely, microbially derived serine proteinases exhibit high esterase activity for N-acyl-L-phenylalanine esters. Furthermore, it has been found that the high esterase activity exhibited for the L-isomer is not inhibited by the presence of the D-isomer. Subjecting N-acyl-D,L-phenylalanine ester to the action of such a proteinase provides a mixture of N-acyl-D-phenylalanine ester and N-acyl-L-phenylalanine. The N-acyl-L-phenylalanine can be readily separated from the mixture by conventional means, for example, by adjusting the pH of an aqueous mixture thereof and extracting with an organic solvent such as chloroform, ethyl acetate, butyl acetate, methylene chloride and the like.

Serine proteinases suitable for use in this invention are derived from microorganisms such as bacteria, fungi and mold. Microbially derived serine proteinases are preferred for use in the process of this invention. These proteinases are relatively inexpensive and commercially available.

An example of preferred serine proteinases for use in this invention are those derived from the bacterial organism *Bacillus subtilis* and termed subtilisins.

A preferred subtilisin of the present invention is the *Bacillus subtilis*-derived Carlsberg strain. The Carlsberg strain employed in accordance with the present invention is a known subtilisin strain, the amino acid sequence of which is described in Smith et al, "The Complete Amino Acid Sequence of Two Types of Subtilisin, BPN′ and Carlsberg," *J. of Biol. Chem.*, Vol. 241, Dec. 25, 1966, at page 5974. This subtilisin strain is characterized by a tyrosine to tryptophan ratio of about 13:1. The reference provides description of the amino acid sequence of the Carlsberg subtilisin.

An X-ray mutated *Bacillus subtilis*-derived subtilisin constitutes another preferred subtilisin of the present invention. This mutation can be effected in accordance with U.S. Pat. No. 3,031,381 issued April 24, 1962, to Minagawa et al by irradiation of a *Bacillus subtilis* organism with X-rays. Subsequent treatment in a conventional manner can be employed to result in the preparation of an enzymatic composition. The patent describes a process whereby an enzymatic composition is produced by subjecting *Bacillus subtilis* to X-rays of an intensity corresponding substantially to 24 to 50 roentgens for an interval of at least half an hour, selecting from the colony thus subject to X-rays a strain identified by cells having hairless, rough, jagged, spotted and dull white characteristics, separating said strain and placing the separated strain in a culture selected from the group consisting of wheat bran and corn meal, maintaining the culture for a period of at least 40 hours while aerating the culture substantially continuously, and drying the culture.

Other examples of suitable serine proteinases for use herein include the following. Serine proteinases derived from *Aspergillus oryzae*. Methods for producing and separating these mold-derived enzymes are known to those skilled in the art. See, for example, Subramamian et al, *Biochemistry*, Vol. 3, No. 12, Pages 1861—74 (1964), and Misaki et al, *Agr. Biol. Chem.*, Vol. 34, No. 9, Pages 1383—92 (1970). Serine proteinase derived from *Streptomyces griseus* (ATCC 3463). Such serine proteinases are available commercially under the tradename "Pronase" from Kaken Chemical Co., Japan. Methods for producing and separating proteinases are known. See, for example, Narahashi et al, *The Journal of Biochemistry*, Vol. 62, No. 6 Pages 633—41 (1967). Serine proteinase derived from *Aspergillus sydowi*. Methods for producing and separating this fungally derived serine proteinase are known. See, for example, Danno et al, *Agr. Biol. Chem.*, Vol. 31, No. 10, Pages 1151—58 (1967).

Other suitable examples of microbially derived serine proteinases are Aspergillus alkaline proteinase (E. C. 3.4.21.15), Alternaria endopeptidase (E. C. 3.4.21.16), Arthrobacter serine proteinase (E. C. 3.4.21.17). These particular enzymes have been identified according to a systematic nomenclature involving an "E. C. number" See "Enzyme Nomenclature", Commission of Biochemical Nomenclature, Elsevier Publishing Company (1973), U.S. Library of Congress Card No. 73—78247.

The action of the proteolytic enzyme on the N-acetyl-D,L-phenylalanine methyl ester is very suitably conducted in an aqueous medium maintained at a pH of from about 5 to 10, preferably from about 7 to about 8, and at a temperature of from about 10° to about 60°C. Preferably, the temperature is maintained in the range of from about 20° to about 40°C.

Because of the high selective esterase activity of the particular proteolytic enzymes employed in this invention toward N-acetyl-L-phenylalanine methyl ester in N-acetyl-D,L-phenylalanine methyl ester mixtures very small amounts of the proteolytic enzyme are required in order to rapidly produce N-acetyl-L-phenylalanine and separate it from N-acetyl-D-phenylalanine methyl ester. For example, aqueous solutions containing from about 0.0005 percent to about 1.0 percent, by weight, preferably from about 0.005 percent to about 0.5 percent, by weight, of enzymes are employed. The amounts of enzyme referred to herein refer to pure crystalline enzyme.

The amount of N-acetyl-D,L-phenylalanine methyl ester employed will generally be at least about 5 percent, by weight, of the aqueous solution. Preferably, larger amounts are employed, for example, amounts up to and exceeding the maximum solubility of the N-acetyl-D,L-phenylalanine methyl ester in the aqueous medium. Preferably, about 10 weight percent of N-acetyl-D,L-phenylalanine methyl ester in the aqueous solution is preferred. Amounts slightly exceeding the maximum solubility can be employed since ase the L-isomer is consumed by the action of the enzyme more will enter solution.

The rate of the action of the enzyme on the material will depend on the concentration of the enzyme and ester in solution. In this regard, N-acetyl-D,L-phenylalanine methyl ester is quite suitable in that it exhibits good solubility in water (about 20 percent by weight at pH 7.5 and 25°C).

Example
*Part A. Preparation of N-acetyl-D,L-phenylalanine methyl ester*

Into a 100 milliliter round bottom flask were placed 10.36 grams (50 millimoles) of N-acetyl-D,L-phenylalanine, 48 grams (1.5 moles) of methanol and 2 grams of sulfuric acid (0.02 moles). The mixture was refluxed for 3 hours. The solvent was then removed under vacuum and the resulting oily residue was taken up in 100 milliliters of ether, washed with 50 milliliters of 5 percent sodium bicarbonate, 50 milliliters of saturated sodium chloride solution, dried over magnesium sulfate, filtered and the solvent removed under water aspirator vacuum to leave an oily residue. The oily residue was titurated with 100 milliliters of petroleum ether and then the petroleum ether was removed with a water aspirator. The oily residue was vacuum dried in 3 minutes. The white mass was broken up and washed with additional petroleum ether, filtered and vacuum dried. Yield of the white product, N-acetyl-D,L-phenylalanine methyl ester, was 75 percent. The melting point was 62° to 64°. The proton NMR and infra-red spectra agreed with the conclusion that the methyl ester of N-acetyl-D,L-phenylalanine was prepared.

Example
*Part B. Separation of N-acetyl-D-phenylalanine methyl ester*

Into a 100 milliliter beaker were placed 2.21 grams (10 millimoles) of N-acetyl-D,L-phenylalanine methyl ester and 19.91 grams of water. The slurry was adjusted to pH 7.5 with 0.2 N sodium hydroxide. Then, 0.01 grams (0.05 weight percent based on the aqueous solution) of purified Carlsberg subtilisin, a serine proteinase commercially available under the tradename "Alcalase" from Novo Industries, Copenhagen, Denmark, was added with stirring. The pH of the solution immediately decreased and was readjusted to pH 7.5 and maintained thereat until enzyme activity ceased after about 46 minutes. A total of 24.3 milliliters of 0.2 $N$ sodium hydroxide was delivered.

The reaction mixture was extracted twice with 100 milliliters of methylene chloride. The organic extracts were combined, dried and the solvent removed under vacuum to yield 1.08 grams of N-acetyl-D-phenylalanine methyl ester. The yield was 97.6 percent, proton NMR confirmed the structure. The optical purity obtained was 98 percent.

The aqueous layer from the extracts was acidified to a pH of 1 by dropwise addition of concentration sulfuric acid. It was then extracted twice with 100 milliliter portions of ethyl acetate. The organic extracts were combined, dried and solvent removed under vacuum to yield 1 gram of N-acetyl-L-phenylalanine for 96.5 percent yield. Proton NMR confirmed the structure and the optical purity was greater than 98 percent of N-acetyl-L-phenylalanine.

Thus, the racemic solution can be separated and substantially pure N-acetyl-D-phenylalanine methyl ester can be obtained. This example represents the comprehensive procedure for obtaining optically pure N-acetyl-D-phenylalanine methyl ester. It should be understood by a skilled artisan that a variety of methods are available for forming N-acetyl-D,L-phenylalanine esters and a variety of methods are also available for separating mixtures of N-acetyl-D-phenylalanine methyl esters and N-acetyl-L-phenylalanine.

The foregoing example can be repeated employing serine proteinases such as subtilisin BPN, BPN' or *Aspergillus oryzae* derived proteinase with similar results.

The N-acetyl-D-phenylalanine ester provided by the process of this invention can be converted to D-phenylalanine in a simple procedure. In one procedure, it can be treated at elevated temperatures with dilute acid; for example, it can be dissolved in 2 $N$ HBr and this solution warmed for a time at 80° to 100°C. Other simple hydrolysis procedures can be employed to convert the N-acetyl-D-phenylalanine methyl ester into the desired D-phenylalanine.

D-phenylalanine has recently been determined to provide significant analgesic action. It is believed to work by inhibiting enzymes responsible for destroying naturally produced, short-acting pain killers. Specifically, D-phenylalanine has been found to be an inhibitor of carboxypeptidase A, and is useful in preventing such enzymes from breaking down enkephalins which are natural analgesics. Tests have shown marked long-term analgesia using D-phenylalanine as an investigational new drug.

As already noted, it has been heretofore recognized that a possibility exists of a preferential enzymatic action on a racemic mixture of derivatives of certain amino acids. For industrial applicability, the mere existence of some selectivity is not enough to assure serious consideration for use. Particularly, where one or both potential end-products are contemplated for dietary or pharmaceutical use, it may be essential to have a particularly high degree of selectivity, otherwise even minor reaction with the less active component will result in a corresponding carry through of its reaction product as an ultimate impurity. Similarly, as a reciprocal expression of the indicated performance factors, it should be possible to selectively react essentially all of one isomer derivatives whereby, of course, a high purity level of the unreacted component is achieved.

The example given above illustrates the impressive attainment of these two performance criteria, as it is seen that both N-acetyl-D-phenylalanine methyl ester and N-acetyl-L-phenylalanine were obtained at 98 percent optical purity, or 99 percent gravimetric purity.

A further factor pertinent to industrial applicability is the rate of reaction which can be obtained. Of course, high selectivity as to one isomer and satisfactorily low selectivity with respect to the other, while essential, would not along make a reaction a commercial possibility. If the reaction rate was inordinately low, of course, extremely long residence times required would adversely affect the economics. As is shown by the example provided, a modest residence time, in which operations, of 46 minutes at ambient temperature, resulted in essentially complete reaction with complete selectivity.

It is of interest to contrast the susceptibility of the N-acetyl-L-phenylalanine methyl ester of this invention with the reaction susceptibility of corresponding derivatives of other L-amino acid derivatives. Data given by Barel (cited above), supplemented by further studies, provide such contrast as tabulated below.

| Substrate N-acetyl-methyl ester of | V max (sec$^{-1}$) |
|---|---|
| L-tyrosine | 1930[1] |
| L-tryptophan | 820[1] |
| L-phenylalanine | 765[1] |
| L-methionine | 523[2] |
| L-valine | 23[1] |
| L-proline | 0[2] |

[1] Reported by Barel.
[2] Separately determined.

As seen from the above, there is profound variation in the rates of reactions with the different amino acid derivatives. It appears that the susceptibility of reaction of the phenylalanine derivative fortuitously is optimum to achieve the desired separation.

Lastly, upon completion of the selective reaction in a common reaction medium, the ease of obtaining a sharp physical separation of the two components is, of course, important. As demonstrated by the example, complete separation was readily achieved by a single and rapid extraction first of the acetyl-D-phenylalanine methyl ester. Subsequently, equally facile recovery was obtained of the N-acetyl-L-phenylalanine.

A contrast is provided by corresponding operations performed to separate the N-acetyl-D,L-methionine methyl ester system. In that situation, after selectively reacting with Carlsberg subtilisin by the same technique as described in the examples herein, it required three successive extractions to obtain satisfactory removal of the unreacted N-acetyl-D-methionine methyl ester. The degree of extraction in the successive steps were

| 1st | 82 percent |
|-----|------------|
| 2nd | 91 percent |
| 3rd | 97 percent. |

As shown by the examples given herein, with the phenylalanine derived system, an approximately 98 percent resolution was accomplished by the one-stage initial extraction separation with methylene chloride.

The selective reaction and subsequent separations disclosed herein can be part of plural-step processes which, in substance, provide for the conversion of racemic mixture of D,L-phenylalanine or derivatives into essentially optically pure D- or L-forms. If it is desired to obtain essentially only the L-phenylalanine structure, the N-acetyl-D-phenylalanine methyl ester is racemized by thermal treatment in presence of an alkaline catalyst, to a 50—50 D,L system and it is merely recycled as a portion of the feed mixture. When it is desired to obtain the D-phenylalanine structure in the product, the N-acetyl-L-phenylalanine is again converted to the methyl ester form and racemized to provide a portion of the feed stream to the selective reaction step.

## Claims

1. A process for separation of D,L-phenylalanine characterized by providing an aqueous solution of a mixture of N-acetyl-D-phenylalanine methyl ester and N-acetyl-L-phenylalanine methyl ester subjecting said mixture at a pH of 5 to 10 to the action of a proteolytic enzyme which is a microbially derived serine proteinase, for a time sufficient to convert the N-acetyl-L-phenylalanine methyl ester to N-acetyl-L-phenylalanine, then separating the N-acetyl-L-phenylalanine from the unreacted N-acetyl-D-phenylalanine methyl ester.

2. A process for the conversion of a racemic mixture of D,L-phenylalanine to D-phenylalanine characterized by the steps of

a. the selective conversion of N-acetyl-L-phenylalanine methyl ester to N-acetyl-L-phenylalanine and separation thereof as defined by Claim 1,

b. the racemization and esterification of said separated N-acetyl-L-phenylalanine to a mixture of N-acetyl-L,D-phenylalanine methyl esters, and

c. the recycle of said mixture to the selective conversion step as defined by Claim 1.

3. A process for the conversion of a racemic mixture of D,L phenylalanine to L-phenylalanine characterized by the steps of

a. the selective conversion of N-acetyl-L-phenylalanine methyl ester to N-acetyl-L-phenylalanine and separation thereof as defined by Claim 1,

b. the racemization of the unreacted N-acetyl-D-phenylalanine methyl ester from step a. to a racemic mixture of N-acetyl-D-phenylalanine methyl ester and N-acetyl-L-phenylalanine methyl ester, and

c. the recycle of said racemic mixture to step a.

## Revendications

1. Un procédé pour la séparation de D,L-phénylalanine caractérisé en ce qu'on se procure une solution aqueuse d'un mélange d'ester de méthyle de N-acétyl-D-phénylalanine et d'ester de méthyle de N-acétyl-L-phénylalanine, on soumet ce mélange à un pH de 5 à 10 à l'action d'une enzyme protéolytique qui est une sérine protéinase d'origine microbienne, pendant un temps suffisant pour transformer l'ester de méthyle de N-acétyl-L-phénylalanine en N-acétyl-L-phénylalanine, puis on sépare la N-acétyl-L-phénylalanine de l'ester de méthyle de N-acétyl-D-phénylalanine n'ayant pas réagi.

2. Un procédé pour la transformation d'un mélange racémique de D,L-phénylalanine en D-phénylalanine, caractérisé par les étapes consistant en

a) la transformation sélective de l'ester de méthyle de N-acétyl-L-phénylalanine en N-acétyl-L-phénylalanine et et sa séparation comme défini par la revendication 1,

b) la racémisation et l'estérification de la N-acétyl-L-phénylalanine séparée en un mélange d'esters de méthyle de N-acétyl-L, D-phénylalanine, et

c) le recyclage de ce mélange à l'étape de transformation sélective comme défini par la revendication 1.

3. Un procédé pour la transformation d'un mélange racémique de D,L-phénylalanine en L-phénylalanine, caractérisé par les étapes comprenant

a) la transformation sélective de l'ester de méthyle de N-acétyl-L-phénylalanine en N-acétyl-L-phénylalanine et sa séparation comme défini dans la revendication 1,

b) la racémisation de l'ester de méthyle de N-acétyl-D-phénylalanine n'ayant pas réagi provenant de l'étape a), donnant un mélange racémique d'ester de méthyle de N-acétyl-D-phénylalanine et d'ester de méthyle de N-acétyl-L-phénylalanine, et

c) le recyclage de ce mélange racémique à l'étape a).

**Patentansprüche**

1. Verfahren zum Trennen von D- und L-Phenylalanin, dadurch gekennzeichnet, daß eine wäßrige Lösung eines Gemisches von N-Acetyl-D-phenylalaninmethylester und N-Acetyl-L-phenylalaninmethylester geschaffen und bei einem ph-Wert von 5 bis 10 der Einwirkung eines proteolytischen Enzyms, das eine mikrobiell erzeugte Serinproteinase ist, so lange unterworfen wird, daß der N-Acetyl-L-phenylalaninmethylester in N-Acetyl-L-phenylalanin umgewandelt wird, und daß danach das N-Acetyl-L-phenylalanin von dem nicht umgesetzten N-Acetyl-D-phenylalaninmethylester abgetrennt wird.

2. Verfahren zum Umwandeln eines racemischen Gemisches von D- und L-Phenylalanin in D-Phenylalanin, gekennzeichnet durch folgende Schritte:

a. Selektive Umwandlung des N-Acetyl-L-phenylalaninmethylesters in N-Acetyl-L-phenylalanin und Abtrennung desselben gemäß Anspruch 1,

b. Racemisierung und Veresterung des abgetrennten N-Acetyl-L-phenylalanins zu einem Gemisch von N-Acetyl-L- und -D-phenylalaninmethylester und

c. Rückführung des Gemisches zu dem selektiven Umwandlungsschritt gemäß Anspruch 1.

3. Verfahren zum Umwandeln eines racemischen Gemisches aus D- und L-Phenylalanin in L-Phenylalanin, gekennzeichnet durch folgende Schritte:

a. Selektive Umwandlung von N-Acetyl-L-phenylalaninmethylester in N-Acetyl-L-phenylalanin und Abtrennung desselben gemäß Anspruch 1,

b. Racemisierung des nach dem Schritt a. vorhandenen, nicht umgesetzten N-Acetyl-D-phenylalaninmethylesters zu einem racemischen Gemisch von N-Acetyl-D-phenyl-alaninmethylester und N-Acetyl-L-phenylalaninmethylester und

c. Rückführung dieses racemischen Gemisches zum Schritt a.